# EUROPÄISCHE PATENTANMELDUNG

(11) **EP 0 816 323 A2**
(43) Veröffentlichungstag der Anmeldung: **07.01.1998**
(21) Anmeldenummer: 97111009.3
(22) Anmeldetag: 02.07.1997
(51) Int. Cl.: C07C 209/26

(54) **Verfahren zur Herstellung primärer und/oder sekundärer Amine aus Oxoverbindungen**

(30) Priorität: 06.07.1996 DE 19627265
(71) Anmelder: Degussa Aktiengesellschaft, 60311 Frankfurt (DE)
(72) Erfinder: Haas, Thomas, Dr., 60316 Frankfurt (DE); Arntz, Dietrich, Dr., 63829 Oberursel (DE); Weber, Karl-Ludwig, Dr., 64807 Dieburg (DE); Hofen, Willi, 63517 Rodenbach (DE); Wieland, Stefan, Dr., 63069 Offenbach (DE)

(57) **Zusammenfassung**

Die Erfindung betrifft ein Verfahren zur Herstellung primärer und/oder sekundärer Amine aus Oxoverbindungen durch katalytische Iminierung der Oxoverbindung mit Ammoniak oder einem primären Amin und nachfolgende Hydrierung.

Erfindungsgemäß erfolgt die Iminierung in Gegenwart eines neuen Iminierungskatalysators, nämlich eines Sulfonatgruppen enthaltenden Organopolysiloxans. Dieser Iminierungskatalysator zeichnet sich durch eine hohe Aktivität aus. Das Verfahren eignet sich insbesondere zur Herstellung von Isophorondiamin aus Isophoronnitril, wobei der Gehalt an Nebenprodukten erniedrigt und die Ausbeute erhöht werden konnten.

## Beschreibung

Die Erfindung betrifft ein Verfahren zur Herstellung primärer und/oder sekundärer eine aus Oxoverbindungen, die gegebenenfalls weitere zur Reduktion befähigte Gruppen enthalten können, durch Iminierung der Oxoverbindung und Hydrierung der Reaktionsprodukte der Iminierung. Die Erfindung richtet sich bevorzugt auf die Herstellung primärer Mono- und Diamine aus Aldehyden und Ketonen und insbesondere auf die Herstellung von Isophorondiamin (= 3-Aminomethyl-3,5,5-trimethyl-cyclohexylamin) aus Isophoronnitril (= 3-Cyano-3,5,5-trimethyl-cyclohexanon).

Es ist bekannt, Oxoverbindungen, etwa Ketone und Aldehyde, durch reduktive Aminierung unter Verwendung von Ammoniak oder einem Monoalkylamin und Wasserstoff in primäre und/oder sekundäre Amine zu überführen. Diese Umsetzung kann in Gegenwart eines einzigen Katalysators, also eines die Iminierung und die sich anschließende Hydrierung katalysierenden Katalysators, oder in Gegenwart von zwei Katalysatoren, wobei der erste die Iminierung und der zweite die Hydrierung katalysiert, durchgeführt werden. In Fällen, in welchen bei einstufiger Fahrweise unter Verwendung eines einzigen Katalysators ein ungenügender Umsatz und/oder unerwünschte Nebenprodukte, etwa der durch direkte Hydrierung der Oxoverbindung erhältliche primäre Alkohol, erhalten werden, kann es vorteilhaft sein, die reduktive Aminierung zweistufig durchzuführen. Zur Herstellung von Isophorondiamin aus Isophoronnitril sind sowohl einstufige Verfahren - siehe zum Beispiel EP-A 0 659 734 - als auch zweistufige Verfahren - siehe zum Beispiel EP-B 0 042 119 - bekannt. Da Isophorondiamin als Epoxidharzhärter und als Rohstoff zur Herstellung von Isophorondiisocyanat möglichst wirtschaftlich und in hoher Reinheit zugänglich sein soll, besteht Bedarf an weiter verbesserten Verfahren.

Gemäß EP-Patent 0 042 119 wird in der gattungsgemäßen Umsetzung von Isophoronnitril zu Isophorondiamin als Iminierungskatalysator ein anorganischer oder organischer Ionenaustauscher in der Ammoniumform eingesetzt. Nachteil der in diesem Dokument genannten Iminierungskatalysatoren, ausschließlich substantiiert werden nur organische Ionenaustauscher, ist deren thermische und gegebenenfalls mechanische Empfindlichkeit.

Um die zuletzt genannten Nachteile zu beheben, wurde im EP-Patent 0 449 089 vorgeschlagen, acide Metalloxide, insbesondere Al₂O₃, TiO₂, SiO₂ und ZrO₂, als Iminierungskatalysator zu verwenden und die Umsetzung in zwei voneinander getrennten Reaktionsräumen, die Iminierung bei 20 bis 150 °C und 15 bis 200 bar und die Hydrierung bei 60 bis 150 °C und 50 bis 300 bar, durchzuführen. Es wurde bei der Nacharbeitung des Verfahrens festgestellt, daß der Anteil an gebildeten Hydroxyaminen, dies sind das cis- und trans-Isomere von Isophoronaminoalkohol (= 3-Aminomethyl-3,5,5-trimethyl-cyclohexanol), sehr hoch ist, so daß ein erhöhter Reinigungsaufwand erforderlich ist.

Bei der Herstellung von Isophorondiamin gemäß DE-Patentanmeldung 195 40 191.3 unter Verwendung eines speziellen Cobalt-Hydrierkatalysator (nach Raney) erwies sich der zusätzliche Einsatz eines Iminierungskatalysators als notwendig, um den Anteil an Hydroxyaminen und anderen unerwünschten Nebenprodukten gering zu halten. Unter Verwendung einer Katalysatorkombination aus Iminierungskatalysator und dem Cobalt-Hydrierkatalysator zur Reduktion der Iminogruppe und der Nitrilgruppe wird Isophorondiamin in sehr guter Ausbeute und hoher Reinheit gewonnen.

Auch die EP-A 0 623 585 lehrt ein zweistufiges Verfahren zur reduktiven Aminierung von Ketonen, wie Isophoronnitril. Iminierungskatalysator ist Aktivkohle, Hydrierkatalysator ein Cobaltkatalysator, enthaltend ein Erdalkalimetallcarbonat und/oder Lanthanoxid. Aus der beispielhaft angegebenen Menge Aktivkohle ermittelt sich ein relativ hohes Katalysatorvolumen im Verhältnis zu umgesetztem (97,7 %) Isophoronnitril. Dies führt zu erhöhten Reaktorkosten. Nachteilig an diesem Verfahren sind ferner der unvollständige Umsatz sowie der hohe Druck.

Auch unter Verwendung trägergebundener Heteropolysäuren als Iminierungskatalysator gemäß DE-A 44 26 472 müssen ein hohes Volumen an Iminierungskatalysator, bezogen auf umgesetztes Isophoronnitril, ein hoher Druck (238 bar) und zudem eine relativ hohe Iminierungstemperatur (70 °C) angewandt werden. Hieraus folgt die Gefahr begrenzter Standzeit des Katalysators durch Desaktivierung.

Aufgabe der vorliegenden Erfindung ist demgemäß, ein weiteres Verfahren zur reduktiven Aminierung von Oxoverbindungen, insbesondere Isophoronnitril, unter Verwendung eines Iminierungs- und eines Hydrierkatalysators aufzuzeigen, das die Nachteile der vorbekannten Verfahren nicht aufweist. Insbesondere sollte das Verfahren in der Iminierungsstufe kontinuierlich bei möglichst geringer Reaktionstemperatur durchführbar sein und bei gegebenem Katalysatorvolumen zu einer höheren Umsetzungsgeschwindigkeit und zu höherem Umsatz und damit geringerem Anteil an Hydroxyaminen im Isophorondiamin-Rohprodukt führen als vorbekannte Systeme.

Gefunden wurde ein Verfahren zur Herstellung primärer und/oder sekundärer Amine aus Oxoverbindungen, die gegebenenfalls weitere zur Reduktion befähigte Gruppen enthalten können, insbesondere zur Herstellung von Isophorondiamin aus Isophoronnitril, wobei man die Oxoverbindung in Gegenwart eines Iminbildungskatalysators in An- oder Abwesenheit eines organischen Lösungsmittels mit überschüssigem Ammoniak zwecks Herstellung primärer Amine oder mit einem niederen Monoalkylamin zwecks Herstellung sekundärer Amine iminiert und die entstandenen Reaktionsprodukte in Gegenwart eines Hydrierkatalysators bei einer Temperatur im Bereich von 20 bis 250 °C und einem Druck im Bereich von 0,5 bis 25 MPa mit Wasserstoff hydriert, das dadurch gekennzeichnet ist, daß man als Iminierungskatalysator ein gegen An- oder Auflösung im Reaktionsmedium beständiges, Sulfonatgruppen enthaltendes Organopolysiloxan verwendet.

Die Unteransprüche richten sich auf bevorzugte Ausführungsformen des Verfahrens.

Das erfindungsgemäße Verfahren läßt sich zur Herstellung primärer und/oder sekundärer Amine aus Oxoverbindungen, worunter wie üblich Carbonylgruppen enthaltende Verbindungen verstanden werden, einsetzen. Die Oxoverbindungen können eine oder mehrere Carbonylgruppen und zusätzlich weitere reduzierbare Gruppierungen, etwa Nitrilgruppen oder olefinische Doppelbindungen, aufweisen. Das Verfahren richtet sich bevorzugt auf die Herstellung von primären Aminen aus aliphatischen, cycloaliphatischen, aromatischen und heteroaromatischen Aldehyden und Ketonen, wobei hierbei Ammoniak als Iminierungsagens dient. Zur Herstellung sekundärer Amine wird ein niederes Monoalkylamin, insbesondere ein (C₁- bis C₄-)Alkylamin, als Iminierungsagens eingesetzt. Das erfindungsgemäße Verfahren eignet sich insbesondere zur Herstellung primärer Mono- und Diamine aus höher molekularen, kompliziert strukturierten oder polyfunktionellen Carbonylverbindungen, bei welchen die einstufige reduktive Aminierung zu einer ungenügenden Raum-Zeit-Ausbeute, zur Bildung unerwünschter Nebenprodukte oder zur Minderung der Katalysatoraktivität führt. Obgleich sehr unterschiedlich strukturierte Oxoverbindungen dem Verfahren zugänglich sind, beispielsweise Benzylamin aus Benzaldehyd, Furfurylamin aus Furfurol und Isophorondiamin aus Isophoronnitril, wird das erfindungsgemäße Verfahren am Beispiel der technisch bedeutsamen Herstellung von Isophorondiamin aus Isophoronnitril dargestellt.

Bei dem erfindungsgemäß zu verwendenden Iminierungskatalysator handelt es sich vorzugsweise um Produkte, wie sie in den US-Patentschriften 4,552,700 und 5,354,831 beschrieben werden. In diesen Produkten ist die Sulfonatgruppe über eine organische Gruppe an das Organopolysiloxangerüst gebunden. Vorzugsweise handelt es sich bei der organischen Gruppe zwischen der Sulfonatgruppe und dem Organopolysiloxangerüst um eine Alkylengruppe mit 1 bis 12 C-Atomen, insbesondere um eine Propylengruppe. Hinsichtlich der Struktur und Variationsmöglichkeiten dieser als Iminierungskatalysator geeigneten Stoffklasse wird ausdrücklich auf die zuvor genannten Patentschriften verwiesen. Ein besonders bevorzugter Katalysator besteht im wesentlichen aus Einheiten der Formel HO₃S-(CH₂)₃-SiO_{3/2} · a SiO_{4/2}, wobei a eine ganze Zahl von 4 bis 20, insbesondere 9, ist. Der Sulfonsäuregehalt bevorzugter Katalysatoren liegt üblicherweise zwischen 0,5 und 1,5 mVal/g des trockenen Katalysators, insbesondere zwischen 0,5 und 1,0 mVal/g. Soweit der Katalysator in einem Festbettreaktor zum Einsatz kommt, wird der Katalysator zweckmäßigerweise in geformter Form, insbesondere kugelförmig, eingesetzt. Ein Katalysator aus im wesentlichen kugelförmigen Teilchen mit einem Durchmesser im Bereich von 0,1 bis 2 mm wird bevorzugt. Ein Verfahren zur Herstellung derartiger geformter Katalysatoren sowie die Stoffdaten derselben sind dem US-Patent 5,354,831 zu entnehmen. Die Formkörper weisen ein hohes Porenvolumen auf; bei den Poren handelt es sich um Meso- und Makroporen.

In der Iminierungsstufe kann der Iminierungskatalysator entweder als Suspensionskatalysator oder in Form eines in einem Reaktor angeordneten Festbetts eingesetzt werden. Vorzugsweise wird der Iminierungskatalysator in einem Festbett angeordnet. Dem Festbettkatalysator kann das umzusetzende Stoffgemisch entweder von unten zugeführt und damit der Reaktor geflutet gehalten werden, oder der Reaktor wird als Rieselbett betrieben, indem das Stoffgemisch oben aufgegeben wird.

In der der Iminierungsstufe nachgeschalteten Hydrierstufe können übliche Hydrierkatalysatoren einsetzt werden, wie sie zur Hydrierung von Iminen und zur Hydrierung von in der Oxoverbindung gegebenenfalls anwesenden anderen reduzierbaren Gruppen allgemein bekannt sind. Bevorzugt wird ein Hydrierkatalysator aus der Reihe cobalt-, nickel-, ruthenium- und/oder anderen edelmetallhaltigen Katalysatoren verwendet. Beispiele solcher Katalysatoren sind in den eingangs zitierten Dokumenten genannt. Auch der Hydrierkatalysator kann in Form eines Suspensions- oder Festbettkatalysators eingesetzt werden. Sofern die Hydrierung unter Verwendung eines Katalysatorfestbetts erfolgt, wird eine Rieselbettfahrweise bevorzugt.

Dem Iminierungsreaktor wird ein Stoffgemisch aus der Oxoverbindung zur Herstellung von Isophorondiamin, also Isophoronnitril, Ammoniak oder im Falle der Herstellung sekundärer Amine ein Monoalkylamin und, gegebenenfalls einem oder mehreren organischen Lösungsmitteln zugeführt. Ammoniak beziehungsweise das Monoalkylamin wird im Überschuß eingesetzt. Vorzugsweise liegt das Mol-Verhältnis von Ammoniak beziehungsweise Monoalkylamin pro Carbonylgruppe der Oxoverbindung im Bereich zwischen etwa 2 und 50. Gemäß einer bevorzugten Ausführungsform enthält das dem Iminierungsreaktor zuzuführende Stoffgemisch auch ein organisches Lösungsmittel, wie insbesondere einen Alkohol oder Ether, wobei Methanol besonders bevorzugt wird. Die Iminierung erfolgt bei einer Temperatur, bei welcher der Iminierungskatalysator gegen Anlösen durch das Reaktionsgemisch stabil ist, üblicherweise unterhalb 100 °C. Bevorzugt wird eine Temperatur im Bereich zwischen 0 und 70 °C, insbesondere zwischen 10 und 30 °C. Zweckmäßigerweise erfolgt die Umsetzung unter dem sich in der geschlossenen Apparatur einstellenden Druck.

Bei der Herstellung von Isophorondiamin enthält das dem Iminierungreaktor vorzugsweise zuzuführende Stoffgemisch - Methanol und 10 bis 40 Gew.-%, insbesondere 10 bis 30 Gew.-% Isophoronnitril sowie 10 bis 40 Gew.-%, vorzugsweise 20 bis 40 Gew.-% Ammoniak. Zusätzlich können diesem Stoffgemisch auch Fraktionen aus der destillativen Aufarbeitung des Isophorondiamins zugesetzt werden, sofern derartige Fraktionen geeignete Rohstoffe zur Bildung von Isophorondiamin enthalten. Zwecks Erhöhung der Gesamtausbeute ist es zweckmäßig, die oberhalb Isophorondiamin siedende Fraktion, welche außer Resten an Isophorondiamin 3,3,5-trimethyl-6-imino-7-aza-bicyclo-[3,2-1]-octan als Hauptprodukt enthält, der Iminierungsstufe zuzuführen; alternativ läßt sich diese Nebenproduktfraktion auch der Hydrierstufe zusetzen.

Das die Iminierungsstufe verlassende Reaktionsgemisch oder ein hieraus hergestelltes, Iminierungsprodukte enthaltendes Stoffgemisch wird der Hydrierstufe zugeführt. Die Hydrierung wird unter an sich bekannten Reaktionsbedingungen durchgeführt. Üblicherweise liegt die Reaktionstemperatur zwischen 20 und 250 °C, meistens oberhalb 50 °C; vorzugsweise erfolgt die Hydrierung bei 50 bis 150 °C, insbesondere 90 bis 130 °C. Die Hydrierung erfolgt in Abhängigkeit vom verwendeten Hydrierkatalysator üblicherweise bei einem Druck von 0,5 bis 25 MPa, insbesondere 3 bis 10 MPa. Im allgemeinen erfolgt die Hydrierung in Gegenwart des oder zumindest eines Teils des in der Iminierungsstufe im Überschuß eingesetzten Ammoniaks beziehungsweise niederen Alkylamins sowie des Lösungsmittels. Das den Hydrierreaktor verlassende Reaktionsgemisch wird in an sich bekannter Weise aufgearbeitet. Diese Aufarbeitung umfaßt üblicherweise Abdestillieren des Ammoniaks beziehungsweise Monoalkylamins und anschließend des Lösungsmittels sowie fraktionierende Destillation des Rohprodukts.

Überraschenderweise ist die Katalysatoraktivität des erfindungsgemäß zu verwendenden Iminierungskatalysators wesentlich größer als diejenige vorbekannter Katalysatoren auf der Basis sulfonatgruppenhaltiger organischer Ionenaustauscher oder acider Metalloxide. Die Aktivität ist auch noch größer als diejenige von Aktivkohle, welche hinsichtlich des Nebenproduktspektrums günstiger abschneidet als die übrigen vorbekannten Iminierungskatalysatoren. Weitere Vorteile der erfindungsgemäß zu verwendenden Iminierungskatalysatoren bestehen darin, daß sie eine hohe Standzeit aufweisen und zudem zu keinem Aktivitätsabfall des nachgeschalteten Hydrierkatalysators führen. Bei der Herstellung von Isophorondiamin aus Isophoronnitril konnte zudem durch Verwendung des erfindungsgemäßen Iminierungskatalysators der Gehalt an Hydroxyaminen, hierunter werden das cis- und trans-Isomere von 3-Aminomethyl-3,5,5-trimethylcyclohexanols verstanden, gegenüber der Verwendung vorbekannter Iminierungskatalysatoren deutlich reduziert werden.

### Beispiel B 1 und Vergleichsbeispiele VB 1 und VB 2

In einem geschlossenen Rührkolben wurden bei 20 °C 20 ml 18 gew.-%ige methanolische Isophoronnitrillösung mit 20 ml 30 gew.-%iger wäßriger Ammoniaklösung versetzt. Hinzugefügt wurden 4 ml Iminierungskatalysator. Mittels eines UV-Detektors wurde die Abnahme der Isophoronnitril-Konzentration gemessen.

Eingesetzt wurde in B 1 ein Sulfonsäuregruppen enthaltendes Organopolysiloxan in Form von hochporösen Kugeln mit einem Durchmesser von 0,1 bis 1,4 mm, hergestellt gemäß US-Patent 5,354,831. Der Katalysator bestand im wesentlichen aus Einheiten der Formel HO₃S-(CH₂)₃-SiO_{3/2} · 9 SiO_{4/2} und wies einen Sulfonsäuregehalt von 0,9 mVal/g trockenen Katalysators auf.

Iminierungskatalysator in VB 1 war ein auf Styrol/Divinylbenzol basierender, Sulfonatgruppen enthaltender organischer Kationenaustauscher (Dowex® 50WX8 der Firma Dow Chemicals).

Iminierungskatalysator in VB 2 war eine Aktivkohle (Norit RAX1 der Firma Norit).

Während mit dem vorbekannten organischen Kationenaustauscher und der Aktivkohle nach 15 Minuten jeweils 35 % des Isophoronnitrils umgesetzt waren, wurde dieser Umsatz unter Verwendung des erfindungsgemäßen sulfonatgruppenhaltigen Organopolysiloxans bereits nach 7,5 Minuten erreicht. Der erfindungsgemäße Iminierungskatalysator ist somit wesentlich aktiver als die vorbekannten Iminierungskatalysatoren.

### Beispiel B2 und Vergleichsbeispiele VB3, VB4, VB5 und VB6

In einer Vorrichtung, umfassend einen Iminierungsreaktor und einen diesem nachgeschalteten Hydrierreaktor, wurde Isophoronnitril in Gegenwart eines Ammoniaküberschusses und Methanol als Lösungsmittel aminierend mit Wasserstoff reduziert.

Als Iminierungsreaktor diente ein mit 15 ml Iminierungskatalysator gefülltes Reaktionsrohr, durch welches ein Gemisch aus Isophoronnitril, Ammoniak und Methanol von unten nach oben gepumpt wurde. Die Temperatur in der Iminierungsstufe wurde auf 25 °C gehalten.

Dem Iminierungsreaktor wurde ein unmittelbar vor dem Reaktoreintritt gemischtes Gemisch aus 52 ml/h Einsatzlösung (24 Gew.-% Isophoronnitril und 76 Gew.-% Methanol) und 28 ml/h flüssigem Ammoniak zugeführt. Der LHSV-Wert im Iminierungsreaktor betrug somit 5,3 h⁻¹.

Als Hydrierreaktor diente ein in Rieselbettfahrweise betriebenes Reaktionsrohr, das 150 ml Hydrierkatalysator enthielt. Eingesetzt wurde ein aktivierter Cobaltkatalysator, hergestellt nach Raney gemäß DE-Patentanmeldung 43 45 265, in Form von Tabletten mit einer Höhe von 5 mm und einem Durchmesser von 3 mm. Der Hydrierreaktor wurde bei 100 °C gehalten, der Druck auf 6 MPa geregelt. Das den Iminierungsreaktor verlassende Gemisch wurde im Gleichstrom mit Wasserstoff auf den Hydrierreaktor gegeben. Der H₂-Gasstrom am Reaktoreingang wurde so eingestellt, daß der gesamte Wasserstoff verbraucht wurde. Die den Reaktor verlassende Flüssigkeit wurde der Analyse zugeführt.

Als Iminierungskatalysator wurde eingesetzt:
- B 2:: erfindungsgemäßer Katalysator wie in B 1
- VB 3:: Titandioxid P25 der Degussa AG in Form von Extrudaten (Durchmesser 1 mm, Höhe 3 bis 4 mm)
- VB 4:: Dowex 50WX8 Kationenaustauscher gemäß VB 1
- VB 5:: Aktivkohle (Norit RAX1) in Form von Strangpresslingen (Durchmesser 1 mm, Höhe 3 bis 4 mm)
- VB 6:: Anorganischer Ionenaustauscher Zeolith ZSM-5 in Form von zylinderförmigen Tabletten (Höhe und Durchmesser etwa 2 mm)

Nach der Analyse (GC-Bestimmung mit internen Standard) des den Hydrierreaktor verlassenden Reaktionsgemischs ergab sich der in der nachstehenden Tabelle angegebene Anteil an Hydroxyaminen, bezogen auf die Summe aller aus Isophoronnitril gebildeten Produkte, also im wesentlichen des Isophorondiamins, der beiden Isomeren Hydroxyamine (cis- und trans-3-Aminomethyl-3,5,5-trimethyl-cyclohexanol) des 3,5,5-Trimethyl-6-imino-7-aza-bicyclo[3,2,1]octans sowie des 2-Aza-4,6,6-trimethyl-bicyclo[3,2,1]octans.

| Beispiel Nr. | Menge Hydroxyamine |
|---|---|
| B 2 | 1,0 Gew.-% |
| VB 3 | 4,0 Gew.-% |
| VB 4 | 3,6 Gew.-% |
| VB 5 | 1,6 Gew.-% |
| VB 6 | 8,5 Gew.-% |

Unter Verwendung des erfindungsgemäßen Iminierungskatalysators werden infolge des höheren Umsatzes in der Iminierungsstufe in geringerem Umfang Hydroxyamine gebildet. Zudem ist im Vergleich zur Verwendung von Aktivkohle die viel höhere Reaktionsgeschwindigkeit hervorzuheben. Festgestellt wurde ferner, daß unter Verwendung von Y-Zeolith sowie mesoporigem MFI-Zeolith ähnlich hohe Mengen an Hydroxyaminen gebildet wurden wie in VB 6 mit Zeolith ZSM5.

### Beispiel B 3 und Vergleichsbeispiel VB 7

Eine Vorrichtung gemäß Beispiel 2 wurde unter den dort angegebenen Betriebsbedingungen 200 Stunden betrieben, wobei einmal der in B 1 definierte erfindungsgemäße Iminierungskatalysator (= B 3) und einmal die in VB 2 angegebene Aktivkohle (= VB 7) eingesetzt wurde.

Die Analyse des Produktgemischs ergab in B 3 auch nach 200 h noch einen Gehalt von 1,0 % Hydroxyamine, bezogen auf die Summe aller aus Isophoron gebildeten Produkte. Die Ausbeute an Isophorondiamin betrug 94,8 bis 95,0 % und blieb während der ganzen Versuchszeit konstant.

Die Analyse des Produktgemischs in VB 7 zeigte, daß der Anteil an Hydroxyaminen innerhalb von 200 h von 1,6 auf 2,0 % anstieg. Außerdem nahm die Hydrieraktivität des dem Iminierungsreaktor nachgeschalteten Hydrierkatalysators während der 200 Stunden deutlich ab; die Ausbeute an Isophorondiamin nahm in dieser Zeit von 94,3 % um 1,5 % ab.

## Patentansprüche

1. Verfahren zur Herstellung primärer und/oder sekundärer Amine aus Oxoverbindungen, die gegebenenfalls weitere zur Reduktion befähigte Gruppen enthalten können, insbesondere zur Herstellung von Isophorondiamin aus Isophoronnitril, wobei man die Oxoverbindung in Gegenwart eines Iminbildungskatalysators in An- oder Abwesenheit eines organischen Lösungsmittels mit überschüssigem Ammoniak zwecks Herstellung primärer Amine oder mit einem niederen Monoalkylamin zwecks Herstellung sekundärer Amine iminiert und die entstandenen Reaktionsprodukte in Gegenwart eines Hydrierkatalysators bei einer Temperatur im Bereich von 20 bis 250 °C und einem Druck im Bereich von 0,5 bis 25 MPa mit Wasserstoff hydriert,
dadurch gekennzeichnet,
daß man als Iminierungskatalysator ein gegen An- oder Auflösung im Reaktionsmedium beständiges, Sulfonatgruppen enthaltendes Organopolysiloxan verwendet.

2. Verfahren nach Anspruch 1,
dadurch gekennzeichnet,
daß man den Iminierungskatalysator in Form von Meso- und Makroporen enthaltenden, im wesentlichen kugelförmigen Teilchen mit einem Durchmesser im Bereich von 0,1 bis 2 mm verwendet.

3. Verfahren nach Anspruch 1 oder 2,
dadurch gekennzeichnet,
daß der Iminierungskatalysator eine Sulfonatgruppen-Kapazität im Bereich von 0,5 bis 1,5 m Val/g, insbesondere 0,75 bis 1,0 mVal/g, aufweist.

4. Verfahren nach einem der Ansprüche 1 bis 3,
dadurch gekennzeichnet,
daß man als Hydrierkatalysator einen oder mehrere Katalysatoren aus der Reihe von cobalt-, nickel-, ruthenium- und/oder anderen edelmetallhaltigen Katalysatoren verwendet.

5. Verfahren nach einem der Ansprüche 1 bis 4,
dadurch gekennzeichnet,
daß man die Iminierung bei einer Temperatur im Bereich von 0 bis 70 °C, insbesondere 10 bis 30 °C, und die Hydrierung bei einer Temperatur im Bereich von 50 bis 150 °C, insbesondere 90 bis 130 °C, durchführt.
